# EUROPEAN PATENT APPLICATION

(11) **EP 4 600 368 A1**
(43) Date of publication of application: **13.08.2025**
(21) Application number: 24305217.2
(22) Date of filing: 09.02.2024
(51) Int. Cl.: C12P 13/02, C12N 9/88

(54) **A METHOD FOR ENZYMATIC PRODUCTION OF AN AMIDE COMPOUND**

(71) Applicant: Adisseo France S.A.S., 92160 Antony (FR); Mitsui Chemicals, Inc., Tokyo 104-0028 (JP)
(72) Inventor: PERUCH, Olivier, 69007 Lyon (FR); BELLIERE-BACA, Virginie, 69390 Millery (FR); ISHIDA, Tsutomu, Toyko 104-0028 (JP); CAPELLE, Nicolas, 69960 Corbas (FR); GELO-PUJIC, Marjana, 69360 Serezin du Rhône (FR)
(74) Representative: Germain Maureau

(57) **Abstract**

The invention concerns a method for preparing a compound of formula (I) and its use for producing methionine and analogs thereof wherein formula (I) is 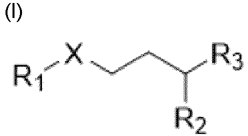 in which
X is selected from S and Se;
R₁ is selected from H, alkyl groups, aryl groups and alkylaryl groups;
R₂ is selected from OH, NR₅R₆ where R₅ and R₆ are identical or different and are selected from H and alkyl groups; and
R₃ is C(O)NR₇R₈ where R₇ and R₈ are identical or different and are selected from H and alkyl groups;
which method comprising the reaction of a compound of formula (II) 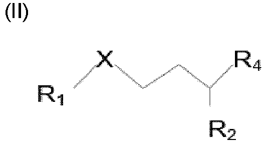 in which
X, R₁ and R₂ are as defined above for the compound of formula (I); and
R₄ is CN;
in the presence of an enzyme having a nitrile hydratase activity and comprising at least an α-subunit and a β-subunit, said α-subunit being defined by SEQ ID No: 1 and said β-subunit being defined by SEQ ID No: 2,
wherein
said α-subunit has at least one mutation selected from the group consisting of Leu6, Ile13, Ala19, Thr36, Asp40, Ala43, Asn48, Arg71, Asp92, Met94, Phe126, Gly148 and Val204, and/or
said β-subunit has at least one mutation selected from the group consisting of Val4, Thr10, Val24, Phe37, Phe41, Met46, Leu48, Phe51, His79, Gln96, Pro107, GIu108, Phe118, Leu127, Arg160, Leu186, Ala200, Gly205, Pro206, Ser212, Tyr215, Ala230 and Ala231,
said amino acid in said position(s) being replaced by anyone of alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, or valine.

## Description

The present invention relates to the manufacture of a sulfur or selenium alpha-fonctionalized amide compound having formula (I) 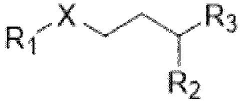 in which
X is selected from S and Se;
R₁ is selected from H, alkyl groups, aryl groups and alkylaryl groups;
R₂ is selected from OH, NR₅R₆ where R₅ and R₆ are identical or different and are selected from H and alkyl groups; and
R₃ is C(O)NR₇R₈ where R₇ and R₈ are identical or different and are selected from H and alkyl groups.

In accordance with an aspect of the invention, it is described a preparation of a precursor involved in the synthesis of methionine, selenomethionine, and analogs thereof, in particular 2-hydroxy-4-methylthiobutyric acid (HMTBA) and 2-hydroxy-4-methylselenobutyric acid (HMSeBA).

The terms "methionine", "selenomethionine", "2-hydroxy-4-methylthiobutyric acid" and "2-hydroxy-4-methylselenobutyric acid" encompass any salt and isomer thereof.

The size of the methionine market is no longer to be presented, particularly in animal nutrition, and its manufacturing processes are still the field of numerous developments, with a view to even more profitable processes, but also less polluting and less energy-consuming. Selenium derivatives of methionine are also constituents of major interest in animal nutrition as well as sulfur/selenium hydroxy- and keto-analogs.

The manufacture of methionine and HMTBA may be operated by various direct or indirect hydrolysis methods of methionine nitrile and 2-hydroxy-4-methylthiobutyronitrile (HMTBN), respectively. This hydrolysis is conventionally carried out by a mineral acid such as hydrochloric acid or sulfuric acid, it may also be implemented in the presence of a catalyst.

Thus, WO00/02852A1 describes the preparation of HMTBA by hydrolysis of HMTBN with sulfuric acid in two steps. According to a first step, there is carried out a hydration reaction of HMTBN into 2-hydroxy-4-methylthio-butyramide (HMTBM) which is thereafter hydrolyzed in a second step into HMTBA.

WO2020/069860A1 discloses the production of methionine comprising the hydrolysis of methionine nitrile in an aqueous mixture comprising a ketone as a carbonyl-containing catalyst and KOH as a basic catalyst and the further hydrolysis of the resulting methionine amide in an acidic, neutral or basic catalyst to form methionine.

The synthesis of HMSeBA is also known. It may in particular be manufactured by acid hydrolysis of 2-hydroxy-4-methylseleno-butyronitrile (HMSeBN) as described in WO2008/049927A1

The manufacture of methionine and HMTBA may also be carried out by enzymatic hydrolysis.

WO98/18941A1 discloses a method for preparing HMTBA from HMTBN comprising contacting HMTBN with a biological material having a nitrilase activity to obtain the ammonium salt of HMTBA and converting the ammonium salt to the corresponding acid.

WO2005/90394A2 pertains to a method for the enzymatic preparation of α-hydroxy- and α-amino-amides such as methionine amide and HMTBM from the corresponding nitriles involving a microbial cyanide-tolerant nitrile hydratase.

However, these biological synthesis of methionine or analogs or precursors thereof can not be performed on an industrial scale in view of the amounts of enzymes that are necessary to reach acceptable yields. Furthermore, they result in the production of high amounts of ammonia thereby causing environment problems.

The present invention provides a solution that overcomes the drawbacks of the prior art with a method for converting a sulfur or selenium alpha-fonctionalized nitrile compound in the corresponding amide compound involving a specific enzyme having a nitrile-hydratase activity. In particular, this method may be practically implemented and it only requires very small amounts of the enzymatic catalyst.

Thus, in accordance with one aspect, the invention relates to a method for preparing a compound of formula (I) 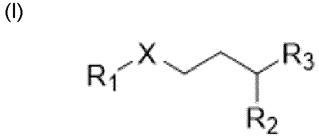 in which
X is selected from S and Se;
R₁ is selected from H, alkyl groups, aryl groups and alkylaryl groups;
R₂ is selected from OH, NR₅R₆ where R₅ and R₆ are identical or different and are selected from H and alkyl groups; and
R₃ is C(O)NR₇R₈ where R₇ and R₈ are identical or different and are selected from H and alkyl groups;
which method comprising the reaction of a compound of formula (II) 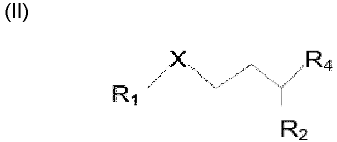 in which
X, R₁ and R₂ are as defined above for the compound of formula (I); and
R₄ is CN;
in the presence of at least one catalyst in an aqueous medium, said catalyst is an enzyme having a nitrile hydratase activity and comprising at least, or consisting of, an α-subunit and a β-subunit, said α-subunit being defined by SEQ ID No: 1 and said β-subunit being defined by SEQ ID No: 2,
wherein
said α-subunit has at least one mutation selected from the group consisting of Leu6, Ile13, Ala19, Thr36, Asp40, Ala43, Asn48, Arg71, Asp92, Met94, Phe126, Gly148 and Val204, and/or
said β-subunit has at least one mutation selected from the group consisting of Val4, Thr10, Val24, Phe37, Phe41, Met46, Leu48, Phe51, His79, Gln96, Pro107, Glu108, Phe118, Leu127, Arg160, Leu186, Ala200, Gly205, Pro206, Ser212, Tyr215, Ala230 and Ala231,
said amino acid in said position(s) being replaced by anyone of alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, or valine.

The enclosed Sequence Listing comprises four sequences, two peptide sequences SEQ ID NO: 1 and SEQ ID NO: 2 for the α-subunit and the β-subunit, respectively,of the wild nitrile hydratase and two nucleotide sequences SEQ ID NO: 3 and SEQ ID NO: 4 encoding SEQ ID NO: 1 and SEQ ID NO: 2, respectively.

The expression "alkyl" refers to a saturated, straight-chain or branched hydrocarbon group having 1-12 carbon atoms, preferably 1-6 carbon atoms, especially 1-4 carbon atoms, for example methyl, ethyl, propyl, isopropyl, n-butyl, isobutyl, tert-butyl.

The expression "aryl" refers to an aromatic group which has one or more rings containing 6-14 ring carbon atoms, preferably 6-10 ring carbon atoms.

The expression "alkaryl" refers to a group which contains both aryl and alkyl groups as defined above.

In one embodiment of the invention, said enzyme comprises, or consists of, an α-subunit being defined by SEQ ID No: 1 and said β-subunit being defined by SEQ ID No: 2, wherein
said α-subunit has at least one mutation selected from the group consisting of Leu6Thr, Leu6Ala, Ile13Leu, Ala19Val, Thr36Met, Thr36Ala, Thr36Trp, Asp40Trp, Ala43Val, Asn48Gln, Arg71His, Asp92Glu, Met94lle, Phe126Tyr, Gly148Asp and Val204Arg; and/or
said β-subunit has at least one mutation selected from the group consisting of Val4Met, Thr10Asp, Val24lle, Phe37Leu, Phe37Val, Phe41lle, Met46Lys, Leu48Val, Phe51Val, His79Asn, Gln96Arg, Pro107Met, Glu108Arg, Glu108Asp, Phe118Val, Leu127Ser, Arg160Trp, Leu186Arg, Ala200Glu, Gly205Val, Pro206Leu, Ser212Tyr, Tyr215Asn, Ala230Glu and Ala231Val.

In a further embodiment, said enzyme comprises, or consists of, an α-subunit being defined by SEQ ID No: 1 and said β-subunit being defined by SEQ ID No: 2, wherein
said α-subunit has the following mutations Leu6Thr, Ala19Val and Phe126Tyr and said β-subunit has the following mutations Met46Lys, Glu108Arg and Ser212Tyr; or
said α-subunit has the following mutations Leu6Thr, Ala19Val and Phe126Tyr and said β-subunit has the following mutations Leu48Val, Glu108Arg and Ser212Tyr; or
said α-subunit has the following mutations Leu6Ala, Ala19Val and Phe126Tyr and said β-subunit has the following mutations Leu127Ser, Arg160Trp and Leu186Arg; or
said α-subunit has the following mutations Leu6Thr, Thr36Met and Phe126Tyr and said β-subunit has the following mutations Thr10Asp, Phe118Val and Ala200Glu; or
said α-subunit has the following mutations Ala19Val, Arg71His and Phe126Tyr and said β-subunit has the following mutations Phe37Leu, Glu108Asp and Ala200Glu; or
said α-subunit has the following mutations Ala19Val, Arg71His and Phe126Tyr and said β-subunit has the following mutations Phe37Val, Glu108Asp and Ala200Glu; or
said α-subunit has the following mutations Thr36Met, Gly148Asp and Val204Arg and said β-subunit has the following mutations Phe41lle, Phe51Val and Glu108Asp; or
said α-subunit has the following mutations Gly148Asp and Val204Arg and said β-subunit has the following mutations Glu108Asp and Ala200Glu; or
said α-subunit has the following mutations Thr36Met, Asp92Glu, Gly148Asp and Val204Arg and said β-subunit has the following mutations Phe41lle, Phe51Val and Glu108Asp; or
said α-subunit has the following mutations Leu6Thr, Ala19Val, Met94lle and Phe126Tyr and said β-subunit has the following mutations Met46Lys, Glu108Arg and Ser212Tyr; or
said α-subunit has the following mutations Ala19Val, Arg71His and Phe126Tyr and said β-subunit has the following mutations Phe37Val, His79Asn, Glu108Asp and Ala200Glu; or
said α-subunit has the following mutations Leu6Thr, Ala19Val and Phe126Tyr and said β-subunit has the following mutations Leu48Val, Gln96Arg, Glu108Arg and Ser212Tyr; or
said α-subunit has the following mutations Ala19Val, Arg71His and Phe126Tyr and said β-subunit has the following mutations Phe37Val, Pro107Met, Glu108Asp and Ala200Glu; or
said α-subunit has the following mutation IIe13Leu and said β-subunit has the following mutations Met46Lys, Gln96Arg, Glu108Arg and Ser212Tyr; or
said α-subunit has the following mutation IIe13Leu, Ala19Val, Arg71His and Phe126Tyr and said β-subunit has the following mutations Phe37Leu, Gln96Arg, Glu108Asp and Ala200Glu; or
said α-subunit has the following mutations Ile13Leu, Thr36Ala and Asn48Gln and said β-subunit has the following mutation Gln96Arg; or
said α-subunit has the following mutations Leu6Thr, Ala19Val and Phe126Tyr and said β-subunit has the following mutations Leu48Val, His79Asn, Glu108Arg, Ser212Tyr and Ala230Glu; or
said α-subunit has the following mutations Thr36Met, Gly148Asp and Val204Arg and said β-subunit has the following mutations Phe41Ile, Phe51Val, Glu108Asp, Pro206Leu and Ala230Glu; or
said α-subunit has the following mutations Thr36Met, Asp92Glu, Gly148Asp and Val204Arg and said β-subunit has the following mutations Val4Met and Pro206Leu; or
said α-subunit has the following mutations Leu6Thr, Ala19Val and Phe126Tyr and said β-subunit has the following mutations Leu48Val, His79Asn, Glu108Arg, Ser212Tyr, Ala230Glu and Ala231Val; or
said α-subunit has the following mutations Leu6Thr, Ala19Val and Phe126Tyr and said β-subunit has the following mutations Val24lle, Leu48Val, His79Asn, Glu108Arg, Ser212Tyr, Ala230Glu and Ala231Val; or
said α-subunit has the following mutations Thr36Trp and Ala43Val and said β-subunit has the following mutation Gly205Val; or
said α-subunit has the following mutations Asp40Trp and Ala43Val and said β-subunit has the following mutation Gly205Val; or
said α-subunit has the following mutations Thr36Trp and Ala43Val and said β-subunit has the following mutation Tyr215Asn; or
said α-subunit has the following mutations Leu6Thr, Thr36Met, Ala43Val and Phe126Tyr and said β-subunit has the following mutations Thr10Asp, Phe118Val, Ala200Glu, Pro206Leu and Ala230Glu; or
said α-subunit has the following mutations Ala43Val and Asp92Glu and said β-subunit has the following Val24lle, Phe41lle, Phe51Val and Glu108Asp; or
said α-subunit has the following mutations Ala19Val, Ala43Val, Arg71His and Phe126Tyr and said β-subunit has the following mutations Phe37Val, His79Asn, Glu108Asp and Ala200Glu.

A method as described above may be implemented in the following conditions which result in the formation of high yields of amide compound while limiting the production of by-products. They may be considered alone or in combination:
it is carried out at a pH of 5-8.5, more preferably at a pH of 7-8; the pH is adjusted by any known pH adjusting agent, for example soda, sulfuric acid, phosphate buffer, gaseous carbon dioxide.
it is carried out at a temperature of 20-50°C, more preferably at a temperature of 20-30°C, at atmospheric pressure;
the amount of compound (II) is 5-20% (m/m) with regard to the aqueous medium;
the weight ratio enzyme : compound (II) is 0.03-0.1;
the reaction time is 1-3 hours.

As previously said, this method may be easily implemented even on an industrial scale. It can be performed in batch or continuously.

The reagents may be add together at the same time. In an embodiment, the nitrile compound is slowly added to an aqueous enzymatic catalyst suspension.

In one embodiment, the heterogeneous enzymatic catalyst can be separated from the reaction medium and it may be recycled upstream from the method. The enzyme may separated by filtration, preferably on an ultrafiltration membrane and/or after treatment with activated carbon followed by filtration.

Therefore, the method of the invention may advantageously comprises a further step consisting of recovering the enzyme and recycling it upstream.

The method of the invention is particularly suitable for the synthesis of methionine, selenomethionine, 2-hydroxy-4-methylthiobutyric acid (HMTBA) and 2-hydroxy-4-methylselenobutyric acid (HMSeBA). Therefore, a further object of the invention is a method for preparing at least one of methionine, selenomethionine, HMTBA and HMSeBA from their corresponding nitrile precursors, namely methionine nitrile, selenomethionine nitrile, HMTBN and HMSeBN, respectively, which implements a method as described above and then converting the resulting amide compounds, namely methionine amide, selenomethionine amide, HMTBM and HMSeBM, respectively, in a conventional manner, in one step or more.

Any method which is well-known from the skilled in the art may be used for this conversion. In one embodiment, it may be performed in the presence of a metallic oxide catalyst, preferably TiOz.

The invention, its advantages and benefits are illustrated in the following examples. It is exemplified for the production of HMTBM and amino-methylthiobutyramide (AMTBM), as represented in the following schemes: 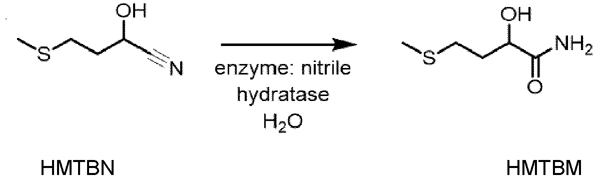 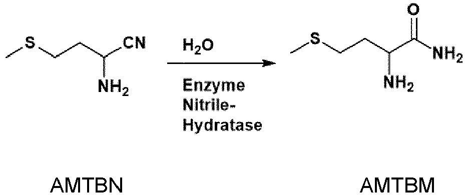 wherein AMTBN may be prepared according to a method as disclosed in WO00/51976A1.

The reaction is monitored by HPLC in a conventional manner. It should be understood that similar conditions apply to obtaining other compounds of formula (II).

The following Examples illustrates a method of the invention for the conversion of HMTBN in HMTBM and AMTBN in AMTBM involving the enzymes as mentioned in the following table, indicating for each, a reference for its production and culture.

**Table 1**

| Example | Mutation in α-subunit | Mutation in β-subunit | Reference for its production |
|---|---|---|---|
| 1 | Leu6Thr, Ala19Val Phe126Tyr | Met46Lys, Glu108Arg Ser212Tyr | WO200456990 (Ex 73 ) |
| 2 | Leu6Thr, Ala19Val, Phe126Tyr | Leu48Val, Glu108Arg, Ser212Tyr | WO200456990 (Ex 74) |
| 3 | Leu6Ala, Ala19Val, Phe126Tyr | Leu127Ser, Arg160Trp, Leu186Arg | WO200456990 (Ex 75) |
| 4 | Leu6Thr, Thr36Met, Phe126Tyr | Thr10Asp, Phe118Val, Ala200Glu | WO200456990 (Ex 76) |
| 5 | Ala19Val, Arg71His, Phe126Tyr | Phe37Leu, Glu108Asp, Ala200Glu | WO200456990 (Ex 77) |
| 6 | Ala19Val, Arg71His, Phe126Tyr | Phe37Val, Glu108Asp, Ala200Glu | WG200456990 (Ex 78) |
| 7 | Thr36Met, Gly148Asp, Val204Arg | Phe41lle, Phe51Val, Glu108Asp | WO200456990 (Ex 79) |
| 8 | Gly148Asp, Val204Arg | Glu108Asp, Ala200Glu | WO200456990 (Ex 80) |
| 9 | Thr36Met, Asp92Glu, Glv148Asp, Val204Arg | Phe41lle, Phe51Val, Glu108Asp | WO201055666 (Ex 2) |
| 10 | Leu6Thr, Ala19Val, Met94lle , Phe126Tyr | Met46Lys, Glu108Arg, Ser212Tyr | WO201055666 (Ex 6) |
| 11 | Ala19Val, Arg71His, Phe126Tyr | Phe37Val, His79Asn, Glu108Asp, Ala200Glu | WO201055666 (Ex 14) |
| 12 | Leu6Thr, Ala19Val, Phe126Tyr | Leu48Val, Gln96Arg, Glu108Arg, Ser212Tyr | WO201055666 (Ex 19) |
| 13 | Ala19Val, Arg71His, Phe126Tyr | Phe37Val, Pro107Met, Glu108Asp, Ala200Glu | WO201055666 (Ex 21) |
| 14 | IIe13Leu | Met46Lys, Gln96Arg, Glu108Arg, Ser212Tyr | WO201055666 (Ex 29) |
| 15 | IIe13Leu, Ala19Val, Arg71His, Phe126Tyr | Phe37Leu, Gln96Arg, Glu108Asp, Ala200Glu | WO201055666 (Ex 30) |
| 16 | IIe13Leu, Thr36Ala, Asn48Gln | Gln96Arg | WO201055666 (Ex 31) |
| 17 | Leu6Thr, Ala19Val, Phe126Tyr | Leu48Val, His79Asn,Glu 108Arg, Ser212Tyr, Ala230Glu | WO201055666 (Ex 46) |
| 18 | Thr36Met, Gly148Asp, Val204Arg | tabPhe41lle, Phe51Val, Glu108Asp, Pro206Leu, Ala230Glu | WO201055666 (Ex 52) |
| 19 | Thr36Met, Asp92Glu, Gly148Asp, Val204Arg | Val4Met, Pro206Leu | WO201055666 (Ex 60) |
| 20 | Leu6Thr, Ala19Val, Phe126Tyr | Leu48Val, His79Asn, Glu108Arg, Ser212Tyr, Ala230Glu, Ala231Val | WO201055666 (Ex 65) |
| 21 | Leu6Thr, Ala19Val, Phe126Tyr | Val24lle, Leu48Val, His79Asn, Glu108Arg, Ser212Tyr, Ala230Glu, Ala231Val | WO201055666 (Ex 71) |
| 22 | Thr36Trp, Ala43Val | Gly205Val | WO2018124247 (Ex 19) |
| 23 | Asp40Trp, Ala43Val | Gly205Val | WO2018124247 (Ex 20) |
| 24 | Thr36Trp, Ala43Val | Tyr215Asn | WO2018124247 (Ex 21) |
| 25 | Leu6Thr, Thr36Met, Ala43Val, Phe126Tyr | Thr10Asp, Phe118Val, Ala200Glu, Pro206Leu, Ala230Glu | WO2018124247 (Ex 24) |
| 26 | Ala43Val, Asp92Glu | Val24lle, Phe41lle, Phe51Val, Glu108Asp | WO2018124247 (Ex 26) |
| 27 | Ala19Val, Ala43Val, Arg71His, Phe126Tvr | Phe37Val, His79Asn, Glu108Asp, Ala200Glu | WO2018124247 (Ex 28) |

### Example 1: Nitrile hydratase 1

### [Production Example 1.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5.1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 1.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 1.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 1.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 2: Nitrile hydratase 2

### [Production Example 2.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 2.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 2.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 2.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 3: Nitrile hydratase 3

### [Production Example 3.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5.1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 3.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 3.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 3.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 4: Nitrile hydratase 4

### [Production Example 4.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 4.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 4.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 4.5]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 5: Nitrile hydratase 5

### [Production Example 5.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 5.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 5.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 5.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 6: Nitrile hydratase 6

### [Production Example 6.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5.1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 6.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 6.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 6.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 7: Nitrile hydratase 7

### [Production Example 7.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 7.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 7.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 7.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 8: Nitrile hydratase 8

### [Production Example 8.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 8.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 8.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 8.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 9: Nitrile hydratase 9

### [Production Example 9.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5.1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 9.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 9.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 9.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 10: Nitrile hydratase 10

### [Production Example 10.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 10.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 10.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 10.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 11: Nitrile hydratase 11

### [Production Example 11.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 11.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 11.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 11.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 12: Nitrile hydratase 12

### [Production Example 12.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5.1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 12.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 12.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 12.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 13: Nitrile hydratase 13

### [Production Example 13.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 13.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 13.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 13.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 14: Nitrile hydratase 14

### [Production Example 14.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 14.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 14.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 14.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 15: Nitrile hydratase 15

### [Production Example 15.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5.1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 15.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 15.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 15.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 16: Nitrile hydratase 16

### [Production Example 16.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 16.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 16.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 16.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 17: Nitrile hydratase 17

### [Production Example 17.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 17.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 17.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 17.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 18: Nitrile hydratase 18

### [Production Example 18.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5.1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 18.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 18.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 18.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 19: Nitrile hydratase 19

### [Production Example 19.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 19.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 19.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 19.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 20: Nitrile hydratase 20

### [Production Example 20.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 20.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 20.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 20.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### < Example 21: Nitrile hydratase 21

### [Production Example 21.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 21.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 21.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 21.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 22: Nitrile hydratase 22

### [Production Example 22.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5.1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 22.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 22.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 22.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 23: Nitrile hydratase 23

### [Production Example 23.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 23.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 23.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 23.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 24: Nitrile hydratase 24

### [Production Example 24.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 24.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 24.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 24.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 25: Nitrile hydratase 25

### [Production Example 25.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5.1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 25.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 25.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 25.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 26: Nitrile hydratase 26

### [Production Example 26.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 26.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 26.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 26.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 27: Nitrile hydratase 27

### [Production Example 27.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5. 1 mL of enzyme suspension containing mutant nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 100% and the yield of HMTBM was 100%.

### [Production Example 27.2]

In a 250 mL stirred reactor (mechanical stirring, double jacket) equipped with a temperature probe, a pH electrode and a heating bath, 130 g of demineralized water and 1 mL of enzyme suspension containing mutant nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 100% and the yield of HMTBM was 100%.

### [Production Example 27.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing mutant nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 100% and the yield of AMTBM was 100%.

### [Production Example 27.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing mutant nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 100% and the yield of AMTBM was 100%.

### Example 28: Wild-type nitrile hydratase

### [Production Example 28.1]

In a 250 mL stirred reactor (mechanical stirring, double jacket) equipped with a temperature probe, a pH electrode and a heating bath, 11 g of HMTBN (95% purity) and 147 g of demineralized water were introduced. Soda 0.1N (~1.5 g) was added to adjust a pH of 5.1 mL of enzyme suspension containing wildtype nitrile hydratase obtained by the above method (142 mg as dry matter) was added afterwards. The mixture was stirred 3 h at 26-29°C, pH was then 5.5. An aliquot was filtered through a 0.2 µm PA filter and analyzed by HPLC. The conversion of HMTBN was 40% and the yield of HMTBM was 40%.

### [Production Example 28.2]

In a 250 mL stirred reactor (mechanical stirring, double jacket) equipped with a temperature probe, a pH electrode and a heating bath, 130 g of demineralized water and 1 mL of enzyme suspension containing wildtype nitrile hydratase obtained by the above method diluted 10 times in water (14 mg as dry matter) were introduced. pH was adjusted around 8.0 with Soda 0.1N. HMTBN (95% purity) was added slowly via syringe pump, 33 g in 8 h at 25-27°C. During this add, soda 0.1N was used to maintain pH between 6.0 and 8.0. An aliquot was filtered through a 0.2 µm PA filter and analyzed by HPLC. The conversion of HMTBN was 20% and the yield of HMTBM was 20%.

### [Production Example 28.3]

In a 250 mL stirred reactor (mechanical stirring, double jacket) equipped with a temperature probe, a pH electrode, a supply of carbon dioxide gas and a heating bath, 12.5 g of AMTBN (80% purity containing residual ammonia) and 100 g of demineralized water were introduced. A pH of 7 was adjusted by CO₂ bubbling. 1 mL of enzyme suspension containing wildtype nitrile hydratase obtained by the above method diluted 10 times (15 mg as dry matter) was added afterwards. The mixture was stirred 2 h at 30°C, pH was maintained around 7.1-7.4 by CO₂ bubbling. Some aliquots were filtered through a 0.2 µm PA filter and analyzed by HPLC. The conversion of AMTBN was 20% and the yield of AMTBM was 20%.

### [Production Example 28.4]

In a 250 mL stirred reactor (mechanical stirring, double jacket) equipped with a temperature probe, a pH electrode, a supply of carbon dioxide gas and a heating bath, 150 g of demineralized water and 1 mL of enzyme suspension containing wildtype nitrile hydratase obtained by the above method diluted 10 times (14.2 mg as dry matter) were introduced. pH was adjusted around 7 with CO₂ bubbling. AMTBN (80% purity containing residual ammonia) was added slowly via a syringe pump, 12 g in 2 h at 30°C with CO₂ bubbling to maintain pH between 7 and 7.5. At the end of the addition, an aliquot was filtered through a 0.2 µm PA filter and analyzed by HPLC. The conversion of AMTBN was 30% and the yield of AMTBM was 30%.

## Claims

1. A method for preparing a compound of formula (I) 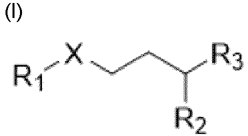 in which
X is selected from S and Se;
R₁ is selected from H, alkyl groups, aryl groups and alkylaryl groups;
R₂ is selected from OH, NR₅R₆ where R₅ and R₆ are identical or different and are selected from H and alkyl groups; and
R₃ is C(O)NR₇R₈ where R₇ and R₈ are identical or different and are selected from H and alkyl groups;
which method comprising the reaction of a compound of formula (II) 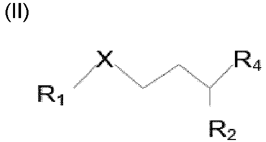 in which
X, R₁ and R₂ are as defined above for the compound of formula (I); and
R₄ is CN;
in the presence of an enzyme having a nitrile hydratase activity and comprising at least an α-subunit and a β-subunit, said α-subunit being defined by SEQ ID No: 1 and said β-subunit being defined by SEQ ID No: 2,
wherein
said α-subunit has at least one mutation selected from the group consisting of Leu6, Ile13, Ala19, Thr36, Asp40, Ala43, Asn48, Arg71, Asp92, Met94, Phe126, Gly148 and Val204, and/or
said β-subunit has at least one mutation selected from the group consisting of Val4, Thr10, Val24, Phe37, Phe41, Met46, Leu48, Phe51, His79, Gln96, Pro107, Glu108, Phe118, Leu127, Arg160, Leu186, Ala200, Gly205, Pro206, Ser212, Tyr215, Ala230 and Ala231,
said amino acid in said position(s) being replaced by anyone of alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, or valine.

2. The method according to claim 1, **characterized in that**
said α-subunit has at least one mutation selected from the group consisting of Leu6Thr, Leu6Ala, Ile13Leu, Ala19Val, Thr36Met, Thr36Ala, Thr36Trp, Asp40Trp, Ala43Val, Asn48Gln, Arg71His, Asp92Glu, Met94lle, Phe126Tyr, Gly148Asp and Val204Arg; and/or
said β-subunit has at least one mutation selected from the group consisting of Val4Met, Thr10Asp, Val24lle, Phe37Leu, Phe37Val, Phe41lle, Met46Lys, Leu48Val, Phe51Val, His79Asn, Gln96Arg, Pro107Met, Glu108Arg, Glu108Asp, Phe118Val, Leu127Ser, Arg160Trp, Leu186Arg, Ala200Glu, Gly205Val, Pro206Leu, Ser212Tyr, Tyr215Asn, Ala230Glu and Ala231Val.

3. The method according to claim 1 **characterized in that**
said α-subunit has the following mutations Leu6Thr, Ala19Val and Phe126Tyr and said β-subunit has the following mutations Met46Lys, Glu108Arg and Ser212Tyr; or
said α-subunit has the following mutations Leu6Thr, Ala19Val and Phe126Tyr and said β-subunit has the following mutations Leu48Val, Glu108Arg and Ser212Tyr; or
said α-subunit has the following mutations Leu6Ala, Ala19Val and Phe126Tyr and said β-subunit has the following mutations Leu127Ser, Arg160Trp and Leu186Arg; or
said α-subunit has the following mutations Leu6Thr, Thr36Met and Phe126Tyr and said β-subunit has the following mutations Thr10Asp, Phe118Val and Ala200Glu; or
said α-subunit has the following mutations Ala19Val, Arg71His and Phe126Tyr and said β-subunit has the following mutations Phe37Leu, Glu108Asp and Ala200Glu; or
said α-subunit has the following mutations Ala19Val, Arg71His and Phe126Tyr and said β-subunit has the following mutations Phe37Val, Glu108Asp and Ala200Glu; or
said α-subunit has the following mutations Thr36Met, Gly148Asp and Val204Arg and said β-subunit has the following mutations Phe41lle, Phe51Val and GIu108Asp; or
said α-subunit has the following mutations Gly148Asp and Val204Arg and said β-subunit has the following mutations GIu108Asp and Ala200Glu; or
said α-subunit has the following mutations Thr36Met, Asp92Glu, Gly148Asp and Val204Arg and said β-subunit has the following mutations Phe41lle, Phe51Val and GIu108Asp; or
said α-subunit has the following mutations Leu6Thr, AIa19Val, Met94lle and Phe126Tyr and said β-subunit has the following mutations Met46Lys, Glu108Arg and Ser212Tyr; or
said α-subunit has the following mutations Ala19Val, Arg71His and Phe126Tyr and said β-subunit has the following mutations Phe37Val, His79Asn, Glu108Asp and Ala200Glu; or
said α-subunit has the following mutations Leu6Thr, Ala19Val and Phe126Tyr and said β-subunit has the following mutations Leu48Val, Gln96Arg, Glu108Arg and Ser212Tyr; or
said α-subunit has the following mutations Ala19Val, Arg71His and Phe126Tyr and said β-subunit has the following mutations Phe37Val, Pro107Met, GIu108Asp and Ala200Glu; or
said α-subunit has the following mutation Ile13Leu and said β-subunit has the following mutations Met46Lys, Gln96Arg, GIu108Arg and Ser212Tyr; or
said α-subunit has the following mutation Ile13Leu, Ala19Val, Arg71His and Phe126Tyr and said β-subunit has the following mutations Phe37Leu, Gln96Arg, GIu108Asp and Ala200Glu; or
said α-subunit has the following mutations Ile13Leu, Thr36Ala and Asn48Gln and said β-subunit has the following mutation Gln96Arg; or
said α-subunit has the following mutations Leu6Thr, Ala19Val and Phe126Tyr and said β-subunit has the following mutations Leu48Val, His79Asn,Glu108Arg, Ser212Tyr and Ala230Glu; or
said α-subunit has the following mutations Thr36Met, Gly148Asp and Val204Arg and said β-subunit has the following mutations Phe41lle, Phe51Val, Glu108Asp, Pro206Leu and Ala230Glu; or
said α-subunit has the following mutations Thr36Met, Asp92Glu, Gly148Asp and Val204Arg and said β-subunit has the following mutations Val4Met and Pro206Leu; or
said α-subunit has the following mutations Leu6Thr, Ala19Val and Phe126Tyr and said β-subunit has the following mutations Leu48Val, His79Asn, Glu108Arg, Ser212Tyr, Ala230Glu and Ala231Val; or
said α-subunit has the following mutations Leu6Thr, Ala19Val and Phe126Tyr and said β-subunit has the following mutations Val24lle, Leu48Val, His79Asn, Glu108Arg, Ser212Tyr, Ala230Glu and Ala231Val; or
said α-subunit has the following mutations Thr36Trp and Ala43Val and said β-subunit has the following mutation Gly205Val; or
said α-subunit has the following mutations Asp40Trp and Ala43Val and said β-subunit has the following mutation Gly205Val; or
said α-subunit has the following mutations Thr36Trp and Ala43Val and said β-subunit has the following mutation Tyr215Asn; or
said α-subunit has the following mutations Leu6Thr, Thr36Met, Ala43Val and Phe126Tyr and said β-subunit has the following mutations Thr10Asp, Phe118Val, Ala200Glu, Pro206Leu and Ala230Glu; or
said α-subunit has the following mutations Ala43Val and Asp92Glu and said β-subunit has the following Val24lle, Phe41lle, Phe51Val and Glu108Asp; or
said α-subunit has the following mutations Ala19Val, Ala43Val, Arg71His and Phe126Tyr and said β-subunit has the following mutations Phe37Val, His79Asn, Glu108Asp and Ala200Glu.

4. The method according to any one of the preceding claims, **characterized in that** it is carried out at a pH of 7-8.5.

5. The method according to any one of the preceding claims, **characterized in that** it is carried out at a temperature of 20-50°C, at atmospheric pressure.

6. The method according to any one of the preceding claims, **characterized in that** the amount of compound (II) is 5-20% (m/m) with regard to the aqueous medium.

7. The method according to any one of the preceding claims, **characterized in that** the weight ratio enzyme : compound (II) is 0.03-0.1.

8. The method according to any one of the preceding claims, **characterized in that** the reaction time is 1-3 hours.

9. The method according to any one of the preceding claims, **characterized in that** it comprises a further step consisting of recovering the enzyme and recycling it upstream.

10. A method for preparing at least one of methionine, selenomethionine, 2-hydroxy-4-methylthiobutyric acid and 2-hydroxy-4-methylselenobutyric acid, **characterized in that** it implements a method according to any one of the preceding claims.

11. The method according to claim 10, wherein the resulting compound (I) is respectively converted in methionine, selenomethionine, 2-hydroxy-4-methylthiobutyric acid and 2-hydroxy-4-methylselenobutyric acid, in the presence of a metallic oxide catalyst, preferably TiOz.
